# EUROPEAN PATENT SPECIFICATION

(11) **EP 3 874 509 B1**
(45) Date of publication and mention of the grant of the patent: **26.08.2026**
(21) Application number: 19806364.6
(22) Date of filing: 31.10.2019
(51) Int. Cl.: G16B 20/00

(54) **METHODS FOR COMPARING EFFICACY OF DONOR MOLECULES**
VERFAHREN ZUM VERGLEICHEN DER WIRKSAMKEIT VON DONORMOLEKÜLEN
MÉTHODES DE COMPARAISON D'EFFICACITÉ DE MOLÉCULES DONNEUSES

(30) Priority: 03.11.2018 US 201862755463 P; 03.04.2019 US 201962828520 P; 12.07.2019 US 201962873264 P
(43) Date of publication of application: 08.09.2021
(73) Proprietor: BlueAllele Corporation, Oakdale, MN 55128 (US)
(72) Inventor: BALTES, Nicholas, Maple Grove, Minnesota 55369 (US)
(74) Representative: Valet Patent Services Limited
(86) International application number: PCT/US2019/059154
(87) International publication number: WO 2020/092763

(56) References cited:
- US-A1- 2017 369 870
- US-A1- 2018 298 377
- XUAN YAO ET AL: "Homology-mediated end joining-based targeted integration using CRISPR/Cas9", CELL RESEARCH, vol. 27, no. 6, 19 May 2017 (2017-05-19), Singapore, pages 801 - 814, XP055516046, ISSN: 1001-0602, DOI: 10.1038/cr.2017.76
- KEVIN R ROY ET AL: "Multiplexed precision genome editing with trackable genomic barcodes in yeast", NATURE BIOTECHNOLOGY, vol. 36, no. 6, 7 May 2018 (2018-05-07), New York, pages 512 - 520, XP055659070, ISSN: 1087-0156, DOI: 10.1038/nbt.4137
- SHAINA N PORTER ET AL: "Lentiviral and targeted cellular barcoding reveals ongoing clonal dynamics of cell lines in vitro and in vivo", GENOME BIOLOGY, BIOMED CENTRAL LTD, vol. 15, no. 5, 30 May 2014 (2014-05-30), pages R75, XP021191459, ISSN: 1465-6906, DOI: 10.1186/GB-2014-15-5-R75
- WADE A REH ET AL: "Gene Targeting by Homologous Recombination", ELS, 15 April 2014 (2014-04-15), Chichester, UK, pages 1 - 10, XP055303559, ISBN: 978-0-470-01617-6, DOI: 10.1002/9780470015902.a0005988.pub2
- ALBERTO MOLINA GIL: "Lentiviral vector packaging cell line development using genome editing to target optimal loci discovered by high- throughput DNA barcoding A thesis submitted for the degree of Doctor of Philosophy 2017", 1 January 2017 (2017-01-01), XP055659099, Retrieved from the Internet <URL:https://discovery.ucl.ac.uk/id/eprint/1573558/1/AMGthesis%20final%20version%20130917.pdf> [retrieved on 20200117]

## Description

### REFERENCE TO RELATED APPLICATIONS

This application claims priority to previously filed and co-pending applications USSN 62/755,463, filed November 3, 2018; USSN 62/828,520, filed April 3, 2019, and USSN 62/873,264 filed July 12, 2019.

### SEQUENCES LISTING

The instant application contains a Sequence Listing which has been submitted in ASCII format via EFS Web. Said ASCII copy, named P12989US03_SEQUENCE LISTING created on October 25, 2019 is named and is 4,096 bytes in size.

### TECHNICAL FIELD

The present document is in the field of genome editing. More specifically, this document relates to the design of donor molecules for gene targeting or targeted insertion.

### BACKGROUND

Gene targeting refers to a process where genomic DNA is modified through homologous recombination. At a minimum, gene targeting requires a user-supplied nucleic acid template, wherein the information from the template is copied into the host's genome at a pre-defined site. Accordingly, gene targeting holds promise for applied application ranging from agriculture to therapeutic diseases. High-throughput cellular barcoding systems have been described for the identification of optimal genomic loci, such as high-transcribing regions, for the site-specific integration of genetic components (see, e.g., Molina Gil, A., "Lentiviral vector packaging cell line development using genome editing to target optimal loci discovered by high-throughput DNA barcoding," PhD Thesis, University College London, 2017). Additionally, massively multiplexed genome editing methods have used barcoded libraries and rare-cutting endonucleases to map the functional effects of genetic mutations in parallel (see, e.g., US Patent Application Publication No. 2017/0369870). However, the technique is still plagued by low efficiencies, often due to the replication status of the target cells (e.g., actively dividing or resting) and DNA repair pathway preferences (e.g., preference for non-homologous end joining instead of homologous recombination). Furthermore, while the aforementioned high-throughput methods focus on identifying genomic positions or studying mutational function, they do not address the impact of the structure of the donor reagent itself on integration frequency. Methods to generate and identify nucleic acid templates optimally suited in their structure for integration through homologous recombination or non-homologous end joining may help advance precise genome modification to applications where efficiency is important.

### SUMMARY

Whereas gene editing holds promise for correcting mutations found in genetic disorders, many challenges remain for creating effective therapies. Of these challenges includes the identification and generation of gene editing reagents that achieve sufficient efficacy for patients to realize benefits. This challenge is exemplified in treatments which require the precise addition or substitution of nucleic acids in a genome. These treatments usually require the delivery of user-supplied templates (i.e., donor molecules) which harbors a cargo flanked by arms of homology. However, the frequency of integration (i.e., gene targeting) is often low, particularly in non-dividing cells. The challenges with identifying effective donor molecules is compounded by observations that: i) small changes within donor molecules can significantly impact integration efficiencies (i.e., changing the length and symmetry of homology arms can impact HR efficiencies), ii) for a single target, the number of potential donor molecules and homology arm structures can be from hundreds to millions or more, iii) comparing efficacy of donors individually can be misleading due to experimental variation between samples, and iv) the efficacy of a specific donor molecule may be different in a conventional cell line as compared to a primary cell line or a cell within an organ in vivo.

The methods described herein provide a way to address the challenges associated with designing donor molecules with optimal structure and efficacy. For example, the methods described herein can reduce the variability caused by testing donors individually (e.g., testing multiple donors at the same time to ensure donors are subject to same experimental variations). Further, the methods provide a way to test a large number of donors in a minimal number of experiments. Also, the methods provide a way to optimize donor molecule structure directly in target cells in vivo (e.g., cells within an organ).

The disclosure herein is based at least in part on the design of a method for evaluating donor molecule integration frequencies by competing donors with different structures against each other in competition assays. The methods are particularly useful in cases where efficiency of gene targeting or targeted insertion is important, including design of therapeutic reagents for treating patients with genetic disorders. Further, the methods permit the high-throughput and direct comparison of numerous donor molecules through competition assays. The methods described herein can be used for applied research (e.g., optimizing gene editing reagents in a therapy for a genetic disorder) or basic research (e.g., determining parameters of homologous recombination or targeted integration efficiencies).

In an embodiment, the document provides a method of identifying the frequency of donor molecule integration into genomic DNA in cells, as defined in claim 1. The donor molecules described herein can have one or two homology arms. Homology arms are nucleic acid sequences and can be referred to as 5' arms or 3' arms or, alternatively, left and right arms. The homology arms can be placed flanking an intervening sequence, either on the 5' or left side of the intervening sequence, or on the right or 3' of the intervening sequence. There may be one arm on the 5' or 3' end, or two arms, one on each the 5' and 3' end. Further, each homology arm will itself have a left or 5' end and a right or 3' end. The intervening sequence may comprise a barcode sequence with or without a cargo. The cargo can be, for example, nucleotides to correct a genetic disorder, the complete or partial coding sequence of a gene, a partial sequence of a gene harboring single-nucleotide polymorphisms relative to the wild type (WT) or altered target, a splice acceptor sequence, a splice donor sequence, a promoter, a terminator, a transcriptional regulatory element, a 2A sequence, purification tags (e.g., glutathione-S-transferase, poly(His), maltose binding protein, Strep-tag, Myc-tag, AviTag, HA-tag, or chitin binding protein) or a reporter gene (e.g., GFP, RFP, lacZ, cat, luciferase, puro, neomycin). Each homology arm can be from 10 to 10,000 bp in length. Differences in homology arms, within donor molecules comprising a single homology arm can include i) one or more additional nucleotides at the 5' end of the homology arm, ii) one or more fewer bases at the 5' end of the homology arm, iii) one or more additional nucleotides at the 3' end of the homology arm, iv) one or more fewer bases at the 3' end of the homology arm, v) the substitution, addition or deletion of nucleic acids within the homology arm (i.e., internal to the 5' and 3' ends), or a combination of i-v. Additionally, in donor molecules comprising two homology arms (a first and second homology arm) the differences can include i) one or more additional nucleotides at the 5' end of the first homology arm, ii) one or more fewer bases at the 5' end of the first homology arm, iii) one or more additional nucleotides at the 3' end of the first homology arm, iv) one or more fewer bases at the 3' end of the first homology arm, v) the substitution, addition or deletion of nucleic acids within the first homology arm (i.e., internal to the 5' and 3' ends), vi) one or more additional nucleotides at the 5' end of the second homology arm, vii) one or more fewer bases at the 5' end of the second homology arm, viii) one or more additional nucleotides at the 3' end of the second homology arm, ix) one or more fewer bases at the 3' end of the second homology arm, x) the substitution, addition or deletion of nucleic acids within the second homology arm (i.e., internal to the 5' and 3' ends), or a combination of i-x. If there is one homologous sequence (i.e., a homology arm), then it will differ from the homologous sequence of the other donor molecules. If there are two homologous arms (i.e., a first and second homology arm), then at least one of homology arms will comprise a difference compared to the other donor molecules. The number of donor molecules comprising different homology sequences delivered to a population of cells can include at least 2 donor molecules, at least 5 donor molecules, at least 10 donor molecules, at least 25 donor molecules, at least 50 donor molecules, at least 100 donor molecules, at least 500 donor molecules, at least 1000 donor molecules, at least 5000 donor molecules, at least 10000 donor molecules, or at least 1000000 donor molecules. The donor molecules are co-delivered with a rare-cutting endonuclease, either in nuclease or nickase format. The rare-cutting endonuclease can be a CRISPR nuclease, a TAL effector nuclease, a meganuclease, or a zinc-finger nuclease. The donor molecules can be single-stranded oligonucleotides, double-stranded oligonucleotides, single-stranded linear DNA, double-stranded linear DNA, single-stranded circular DNA, or double-stranded circular DNA. In embodiments, the donor molecules can be the same format of nucleic acids and can comprise structures having a homologous sequence and a barcode. In one embodiment, the donor molecule can have a structure of 5' - [arm 1] - [barcode] - 3'. In another embodiment, the donor molecules can have a structure of 5' - [arm 1] - [barcode] - [arm 2] - 3'. In another embodiment, the donor molecules can have a structure of 5' - [barcode] - [arm 2] - 3'. In another embodiment, the donor molecules can have a structure of 5' - [arm 1] - [cargo] - [barcode] - [arm 2] - 3'. In another embodiment, the donor molecules can have a structure of 5' - [arm 1] - [barcode] - [cargo] - [arm 2] - 3'. In another embodiment, the donor molecules can have a structure of 5' - [arm 1] - [barcode] - [cargo] - 3'. In another embodiment, the donor molecules can have a structure of 5' - [cargo] - [barcode] - [arm 2] - 3'. In another embodiment, the donor molecules can have a structure of 5' - [arm 1] - [barcode 1] - [cargo] - [barcode 2] - [arm 2] - 3', wherein barcode 1 and barcode 2 are the same barcode or different barcodes within the same donor, but are different barcodes between two donors with differences in homology arms.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In addition, the materials, methods, and examples are illustrative only and not intended to be limiting.

The details of one or more embodiments of the invention are set forth in the description below. Other features, objects, and advantages of the invention will be apparent from the description and from the claims.

### DESCRIPTION OF DRAWINGS

FIG. 1 is a flow chart describing the general steps for determining the relative efficiency of donor molecules within a library.
FIG. 2 is an illustration showing examples for the general structure and composition of donor molecules compatible with the methods described herein.
FIG. 3 is an illustration showing elements that can be present in the arms of donor molecules compatible with the methods described herein.
FIG. 4 is an illustration showing an example of a potential donor molecule that can be used within the methods described herein.
FIG. 5 is an illustration showing the concept of donor molecule competition.
FIG. 6 is an illustration of a single-stranded oligo library of donor molecules for targeting the USH2A c.2299delG site.
FIG. 7 is an illustration showing the target sites for several rare-cutting endonucleases compatible with the donor molecule library targeting the USH2A c.2299delG site.
FIG. 8 is an illustration showing two single-stranded oligonucleotide donors targeting the USH2A gene.
FIG. 9 shows A) the percentage of homologous recombination (HR) using donor oNJB005 or oNJB006 and B) the percentage of each barcode within the sample delivered both oNJB005 and oNJB006.
FIG. 10 is an illustration showing four single-stranded oligonucleotide donors targeting the HBB gene.
FIG. 11 shows A) the percentage of homologous recombination (HR) using donor oNJB001, oNJB002, oNJB003 or oNJB004 and B) the percentage of each barcode within the sample delivered oNJB001, oNJB002, oNJB003 and oN.JB004.

### DETAILED DESCRIPTION

Disclosed herein are methods for testing the integration efficiency of donor molecules. In some embodiments, the methods include delivering two or more donor molecules to a cell or a population of cells, and then assessing the frequency of integration for each donor molecule.

In one embodiment, this document features a method for integrating a nucleic acid sequence into a cell's genome by the delivery of two or more donor molecules. The donor molecule sequence can be compatible with either the homologous recombination pathway or non-homologous end joining pathway. The donor molecules can contain several elements, including sequence that is homologous to a target locus (i.e., facilitates gene targeting through the homologous recombination pathway) or target sites for rare-cutting endonucleases (i.e., facilitates targeted insertion through the non-homologous end joining pathway). The donor molecules can also contain a barcode that is used to identify the original components and elements within individual donor molecules. In one embodiment, the two or more donor molecules can be administered to cells along with a rare-cutting endonuclease that targets a site within the genome. The method can be compatible with the use of any rare-cutting endonuclease, including a CRISPR nuclease, a TAL effector nuclease, or a zinc-finger nuclease. Further, the method can be compatible with a rare-cutting endonuclease in a nickase or nuclease format. In one embodiment, the methods can be used in eukaryotic cells, including plant and mammalian cells. In other embodiments, the donor molecules can further contain a cargo, where the cargo can comprise elements such as the complete or partial coding sequence of a gene, a partial sequence of a gene harboring single-nucleotide polymorphisms relative to the wild type (WT) or altered target, a splice acceptor or splice donor sequence, a promoter, a terminator, a transcriptional regulatory element, a 2A sequence, purification tags (e.g., glutathione-S-transferase, poly(His), maltose binding protein, Strep-tag, Myc-tag, AviTag, HA-tag, or chitin binding protein) or a reporter gene (e.g., GFP, RFP, lacZ, cat, luciferase, puro, neomycin). In some embodiments, the cargo within the two or more donors can be the same nucleic acid sequence. The two or more donor molecules can comprise different sequences (e.g., different homology arm lengths) but they can be targeted to the same gene and compatible with the same rare-cutting endonuclease. In other embodiments, the two or more donors can be in formats including single-stranded oligonucleotides, are double-stranded oligonucleotides, single-stranded linear DNA, double-stranded linear DNA, single-stranded circular DNA, double-stranded circular DNA. The donors can be present on viral or non-viral vectors. In one embodiment, this document provides methods which can be used to identify the frequency of donor molecule integration into genomic DNA in cells, where the method comprises exposing the cells to a plurality of donor molecules, wherein each donor molecule comprises a homology sequence, and at least one barcode, wherein the homology sequence comprises a sequence that is homologous to a target locus within the genomic DNA, wherein the homology sequence for each donor molecule is different; and wherein the at least one barcode for each donor molecule is different. In some embodiments, the cell cultures can be adherent or suspension cell cultures, immortalized cell lines, primary cell lines, or stem cell lines.

In another embodiment, this document provides compositions comprising a plurality of donor molecules, wherein each donor molecule comprises a homology sequence, and at least one barcode, wherein the homology sequence comprises a sequence that is homologous to a target locus within a genome, wherein the homology sequence for each donor molecule is different, and wherein the at least one barcode for each donor molecule is different. In another embodiment, this document provides compositions comprising a plurality of donor molecules, wherein each donor molecule comprises a homology sequence, and at least one barcode, wherein the homology sequence comprises a sequence that is homologous to a target locus within a genome, wherein the homology sequence for each donor molecule is different, and wherein the at least one barcode for each donor molecule is different. In another embodiment, this document provides a method for identifying optimal donor molecule structure for integration into the genomic DNA of cells of an organ, the method comprising identifying the organ; exposing cells within the organ to a plurality of donor molecules, wherein each donor molecule comprises a homology sequence, and at least one barcode, wherein the homology sequence comprises a sequence that is homologous to a target locus within the genomic DNA, wherein the homology sequence for each donor molecule is different; and wherein the at least one barcode for each donor molecule is different. The organ can be an animal organ animal. The animal organ can be removed from the animal. If the organ is removed from the animal, the organ can be prepped for transfection. For example, tissue from the organ can be partially digested and maintained within cell culture before transfection. Alternatively, tissue from the organ can be transfected by direct injection with a solution comprising donor molecules. The animal organ can be transfected in vivo. For example, donor molecules can be delivered systemically with carriers such as lipid nanoparticles. Cells from the transfected organ can be assessed for barcode frequencies. Cells or tissue from the organ can be used for nucleic acid purification.

In another embodiment, this document provides a method for identifying optimal donor molecule structure for the integration into the genomic DNA of cells of a patient, the method comprising identifying the patient; exposing cells from the patient to a plurality of donor molecules, wherein each donor molecule comprises (i) a homology sequence, and (ii) at least one barcode, wherein the homology sequence comprises a sequence that is homologous to a target locus within the genomic DNA, wherein the homology sequence for each donor molecule is different; and wherein the at least one barcode for each donor molecule is different. In one embodiment, the donor molecules described herein can be delivered to cells from a human patient. The cells can be obtained from methods such as a biopsy.

In another embodiment, this document provides a method for identifying the frequency of donor molecule integration into genomic DNA in cells, where the method includes exposing the cells to a plurality of donor molecules, wherein each donor molecule comprises a homology sequence, and at least one barcode, where the homology sequence comprises sequence that is homologous to a target locus within the genomic DNA, and wherein the homologous sequence for at least two of the donor molecules is different, and wherein at least one barcode for the said at least two donor molecules is different. For example, a plurality of donor molecules comprising different homology arms and different barcodes can be generated. The plurality, for example, can be a minimum of two donor molecules. In addition to this plurality of donor molecules, additional donor molecules can be added in addition to the plurality of donor molecules.

In another embodiment, this document features a method to identify the frequency of donor molecule integration into genomic DNA in cells, where the method includes exposing the cells to a plurality of donor molecules, wherein each donor molecule comprises a homology sequence, and at least one barcode, wherein the at least one barcodes are different from the barcodes of the other donor molecules, and wherein each donor molecule is harbored on a different format of DNA or vectors compared to the other donor molecules. For example, the plurality of donor molecules comprising different barcodes and different formats can include a first donor as single-stranded DNA and a second donor, with the same homologous sequence but different barcode, as double-stranded DNA. The plurality, for example, can be a minimum of two donor molecules. In addition to this plurality of donor molecules, additional donor molecules can be added to the plurality of donor molecules. Also, a donor molecule with the same format but a different barcode, as compared to one of the donors within the plurality of donor molecules, can be added in addition to the plurality of donor molecules.

In an aspect, including in any of the aforementioned aspects or embodiments, this document provides methods for determining the frequency of integration of each barcode into the genomic DNA (e.g., through sequencing of genomic DNA or RNA). In an aspect, including in any of the aforementioned aspects or embodiments, the methods and compositions described in this document can use donor molecules having at least one homology arm (e.g., one homology arm or two homology arms).

In an aspect, including in any of the aforementioned aspects or embodiments, the methods and compositions described in this document can use donor molecules having a cargo sequence, where the cargo can comprise elements such as the complete or partial coding sequence of a gene, a partial sequence of a gene harboring single-nucleotide polymorphisms relative to the WT or altered target, a splice acceptor or splice donor sequence, a promoter, a terminator, a transcriptional regulatory element, a 2A sequence, purification tags (e.g., glutathione-S-transferase, poly(His), maltose binding protein, Strep-tag, Myc-tag, AviTag, HA-tag, or chitin binding protein) or a reporter gene (e.g., GFP, RFP, lacZ, cat, luciferase, puro, neomycin). In some cases, the plurality of donor molecules can comprise the same cargo, but have different barcodes and different homology arms. In an aspect, including in any of the aforementioned aspects or embodiments, this document provides methods and compositions for optimizing donor molecule structure. The plurality of donor molecules described in this document can comprise at least two donor molecules, at least five donor molecules, at least ten donor molecules, at least twenty-five donor molecules, at least fifty donor molecules, at least one hundred donor molecules, at least one-thousand donor molecules, two to ten thousand donor molecules, or ten thousand to one million donor molecules.

In an aspect, including in any of the aforementioned aspects or embodiments, the plurality of donor molecules are delivered to cells along with a rare-cutting endonuclease. In another embodiment, the rare-cutting endonuclease can be stably integrated into the cell's genome. The rare-cutting endonuclease can have an inducible promoter. The rare-cutting endonuclease can be a CRISPR nuclease, a TAL effector nuclease, or a zinc-finger nuclease. In some aspects, the rare-cutting endonuclease can be delivered as protein, RNA, DNA, or an RNA/protein mixture. In other aspects, the rare-cutting endonuclease can be a nuclease which cleaves both strands of a target DNA, or a nickase, which cleaves one strand of a target DNA. In an aspect, including in any of the aforementioned aspects or embodiments, the plurality of donor molecules can be delivered to cells, including mammalian cells or plant cells. In an aspect, including in any of the aforementioned aspects or embodiments, the plurality of donor molecules are targeted to a genomic DNA sequence within the same gene. In an aspect, including in any of the aforementioned aspects or embodiments, the plurality of donor molecules can be single-stranded oligonucleotides, double-stranded oligonucleotides, single-stranded linear DNA, double-stranded linear DNA, single-stranded circular DNA, double-stranded circular DNA, or a mixture of single-stranded oligonucleotides, double-stranded oligonucleotides, single-stranded linear DNA, double-stranded linear DNA, single-stranded circular DNA, or double-stranded circular DNA. In an aspect, including in any of the aforementioned aspects or embodiments, the plurality of donor molecules can be harbored on viral vectors, including of retroviral, adenoviral, adeno-associated vectors (AAV), herpes simplex, pox virus, hybrid adenoviral vector, epstein-bar virus, lentivirus, or herpes simplex virus. In an aspect, including in any of the aforementioned aspects or embodiments, the plurality of donor molecules can be harbored on non-viral vectors. The non-viral vectors can be delivered with a reagent including lipids, calcium phosphate, cationic polymers, DEAE-dextran, dendrimers, polyethylene glycol (PEG) cell penetrating peptides, gas-encapsulated microbubbles or magnetic beads.

In an aspect, including in any of the aforementioned aspects or embodiments, the plurality of donor molecules can further comprise single-nucleotide polymorphisms that prevent binding or cleavage by a rare-cutting endonuclease. In an aspect, including in any of the aforementioned aspects or embodiments, the plurality of donor molecules can be delivered to cells within an organ. The cells can be delivered in vivo to cells within an organ. Alternatively, the plurality of donor molecules can be delivered to cells from an organ that was extracted from an animal. The organ can be from an animal. The organ can be from a mammal. The organ can be from a human. The organ can be from mice, rats, hamsters, gerbils, guinea pigs, cats, dogs, rabbits, hedgehogs, horses, goats, sheep, swine, llamas, alpacas, cattle, capuchin monkeys, chimpanzees, lemurs, macaques, marmosets, tamarins, spider monkeys, squirrel monkeys, or vervet monkeys.

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. See, for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P. M. Wassarman and A. P. Wolffe, eds.), Academic Press, San Diego, 1999; and METHODS IN MOLECULAR BIOLOGY, Vol. 119, "Chromatin Protocols" (P. B. Becker, ed.) Humana Press, Totowa, 1999.

As used herein, the terms "nucleic acid" and "polynucleotide," can be used interchangeably. Nucleic acid and polynucleotide can refer to a deoxyribonucleotide or ribonucleotide polymer, in linear or circular conformation, and in either single- or double-stranded form. These terms are not to be construed as limiting with respect to the length of a polymer. The terms can encompass known analogues of natural nucleotides, as well as nucleotides that are modified in the base, sugar and/or phosphate moieties.

The terms "polypeptide," "peptide" and "protein" can be used interchangeably to refer to amino acid residues covalently linked together. The term also applies to proteins in which one or more amino acids are chemical analogues or modified derivatives of corresponding naturally-occurring amino acids.

The terms "operatively linked" or "operably linked" are used interchangeably and refer to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in cis with a coding sequence, but need not be directly adjacent to it. For example, an enhancer is a transcriptional regulatory sequence that is operatively linked to a coding sequence, even though they are not contiguous.

As used herein, the term "cleavage" refers to the breakage of the covalent backbone of a nucleic acid molecule. Cleavage can be initiated by a variety of methods including, but not limited to, enzymatic or chemical hydrolysis of a phosphodiester bond. Cleavage can refer to both a single-stranded nick and a double-stranded break. A double-stranded break can occur as a result of two distinct single-stranded nicks. Nucleic acid cleavage can result in the production of either blunt ends or staggered ends. In certain embodiments, rare-cutting endonucleases are used for targeted double-stranded or single-stranded DNA cleavage.

An "exogenous" molecule can refer to a small molecule (e.g., sugars, lipids, amino acids, fatty acids, phenolic compounds, alkaloids), or a macromolecule (e.g., protein, nucleic acid, carbohydrate, lipid, glycoprotein, lipoprotein, polysaccharide), or any modified derivative of the above molecules, or any complex comprising one or more of the above molecules, generated or present outside of a cell, or not normally present in a cell. Exogenous molecules can be introduced into cells. Methods for the introduction of exogenous molecules into cells can include lipid-mediated transfer, electroporation, direct injection, cell fusion, particle bombardment, calcium phosphate co-precipitation, DEAE-dextran-mediated transfer and viral vector-mediated transfer.

An "endogenous" molecule is a small molecule or macromolecule that is present in a particular cell at a particular developmental stage under particular environmental conditions. An endogenous molecule can be a nucleic acid, a chromosome, the genome of a mitochondrion, chloroplast or other organelle, or a naturally-occurring episomal nucleic acid. Additional endogenous molecules can include proteins, for example, transcription factors and enzymes.

As used herein, a "gene," refers to a DNA region encoding that encodes a gene product, including all DNA regions which regulate the production of the gene product. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

An "endogenous gene" refers to a DNA region normally present in a particular cell that encodes a gene product as well as all DNA regions which regulate the production of the gene product.

"Gene expression" refers to the conversion of the information, contained in a gene, into a gene product. A gene product can be the direct transcriptional product of a gene. F'or example, the gene product can be, but not limited to, mRNA, tRNA, rRNA, antisense RNA, ribozyme, structural RNA, or a protein produced by translation of an mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Encoding" refers to the conversion of the information contained in a nucleic acid, into a product, wherein the product can result from the direct transcriptional product of a nucleic acid sequence. For example, the product can be, but not limited to, mRNA, tRNA, rRNA, antisense RNA, ribozyme, structural RNA, or a protein produced by translation of an mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

A "target site" or "target sequence" or "target locus" for a rare-cutting endonuclease defines a region of a nucleic acid to which a rare-cutting endonuclease molecule will bind, provided sufficient conditions for binding exist. A "target site" or "target sequence" or "target locus" for a donor molecule defines a region of a nucleic acid to which a donor molecule is targeted. The donor molecule can be targeted to a region of a nucleic acid by i) comprising homologous sequence, wherein the homologous sequence can facilitate integration through homologous recombination, or ii) by codelivering a rare-cutting endonuclease which can facilitate integration of the donor molecule through non-homologous end joining.

As used herein, the term "recombination" refers to a process of exchange of genetic information between two polynucleotides. The term "homologous recombination" or "HR" refers to a specialized form of recombination that can take place, for example, during the repair of double-strand breaks. Homologous recombination requires nucleotide sequence homology present on a donor molecule. The donor molecule can be used by the cell as a template for repair of a double-strand break. Information within the donor molecule that differs from the genomic sequence at or near the double-strand break can be stably incorporated into the cell's genomic DNA. Alternatively, a donor molecule can comprise little to no homology to the genomic target site, but can harbor elements that facilitate integration into the genome by the non-homologous end joining pathway. These elements can include exposed single stranded or double-stranded DNA ends, or target sites for cleavage by a rare-cutting endonuclease.

The term "donor molecule integration" refers to the process where all or part of the donor molecule is transferred to the genome, resulting in an addition of one or more nucleic acids within the target site, a subtraction of one or more nucleic acids from the target site, or substitution of one or more nucleic acids within the target site, or any combination of an addition of one or more nucleic acids within the target site, a subtraction of one or more nucleic acids from the target site, and a substitution of one or more nucleic acids within the target site.

The term "homologous" as used herein refers to a sequence of nucleic acids or amino acids having similarity to a second sequence of nucleic acids or amino acids. In some embodiments, the homologous sequences can have at least 80% sequence identity (e.g., 81%, 85%, 90%, 95%, 96%, 97%, 98%, or 99% sequence identity) to one another.

The term "homology sequence" refers to a sequence of nucleic acids that comprises homology to a second nucleic acid. Homology sequence, for example, can be present on a donor molecule as an "arm of homology" or "homology arm." A homology arm can be a sequence of nucleic acids within a donor molecule that facilitates homologous recombination with the second nucleic acid. As defined herein, the homology arm can also be referred to as an "arm". In a donor molecule with two homology arms, the homology arms can be referred to as "arm 1" and "arm 2."

The term "different" when referring to the homology sequence or homology arms present on donor molecules refers to the variation in nucleic acids within the homology sequence or homology arms between the donor molecules. For example, in donor molecules comprising a single homology arm, the difference can include i) one or more additional nucleotides within the 5' end of the homology arm, ii) one or more fewer bases within the 5' end of the homology arm, iii) one or more additional nucleotides within the 3' end of the homology arm, iv) one or more fewer bases within the 3' end of the homology arm, v) the substitution, addition or deletion of nucleic acids within the homology arm (i.e., internal to the 5' and 3' ends), or a combination of i-v. Additionally, and for example, in a donor molecule comprising two homology arms (a first and second homology arm) the difference can include i) one or more additional nucleotides within the 5' end of the first homology arm, ii) one or more fewer bases within the 5' end of the first homology arm, iii) one or more additional nucleotides within the 3' end of the first homology arm, iv) one or more fewer bases within the 3' end of the first homology arm, v) the substitution, addition or deletion of nucleic acids within the first homology arm (i.e., internal to the 5' and 3' ends), vi) one or more additional nucleotides within the 5' end of the second homology arm, vii) one or more fewer bases within the 5' end of the second homology arm, viii) one or more additional nucleotides within the 3' end of the second homology arm, ix) one or more fewer bases within the 3' end of the second homology arm, x) the substitution, addition or deletion of nucleic acids within the second homology arm (i.e., internal to the 5' and 3' ends), or a combination of i-x.

The term "cargo" refers to a nucleic acid molecule which can be integrated at a target locus with the host DNA.

The term "barcode" when described within a donor molecule refers to a sequence of nucleic acids that can be used to identify the original structure of a donor molecule. In a mixture with a plurality of donor molecules with different homology arms, the barcode for each of the donor molecules can be different, and after integration of the barcode in the host's DNA, the barcode can be used to determine the original structure of the donor molecule. The length of the barcode can be the same as the barcodes on the other donor molecules, but the sequence can be different compared to the barcodes on the other donor molecules. The length of the barcode can be the different then the barcodes on the other donor molecules, and the sequence can be different compared to the barcodes on the other donor molecules.

As described herein, "WT" or "wild type" nucleic acid refers to the sequence of the nucleic acid that is the most common in a population.

The percent sequence identity between a particular nucleic acid or amino acid sequence and a sequence referenced by a particular sequence identification number is determined as follows. First, a nucleic acid or amino acid sequence is compared to the sequence set forth in a particular sequence identification number using the BLAST 2 Sequences (Bl2seq) program from the stand-alone version of BLASTZ containing BLASTN version 2.0.14 and BLASTP version 2.0.14. This stand-alone version of BLASTZ can be obtained online at fr.com/blast or at ncbi.nlm.nih.gov. Instructions explaining how to use the Bl2seq program can be found in the readme file accompanying BLASTZ. Bl2seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. For example, the following command can be used to generate an output file containing a comparison between two sequences: C:\Bl2seq -i c:\seq1.txt -j c:\seq2.txt -p blastn -o c:\output.txt -q -1 -r 2. To compare two amino acid sequences, the options of Bl2seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\Bl2seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (e.g., 100 consecutive nucleotides or amino acid residues from a sequence set forth in an identified sequence), followed by multiplying the resulting value by 100. The percent sequence identity value is rounded to the nearest tenth.

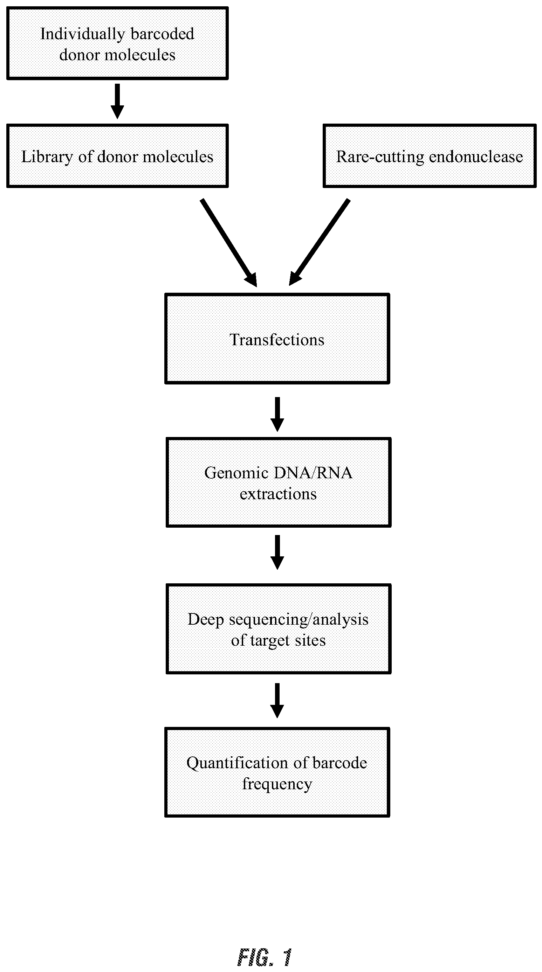

In one embodiment, this document features a method for determining the relative integration frequency of donor molecules. The method can include creating two or more donor molecules, wherein the individual donor molecules harbor one or more unique barcodes (FIG. 1). The barcoded donor molecules are then combined to generate a library of donor molecules. The library can be a mixture of two or more donor molecules at certain ratios. The library can then be combined with one or more rare-cutting endonucleases (for example, a CRISPR Cas nuclease or nickase) in any format (protein, RNA, DNA, or a mixture of protein, RNA or DNA) and transfected into cells. Genomic DNA from the transfected cells can be analyzed for integration of the donor molecules. The frequency of the unique barcodes within the anticipated target site can be used to determine the relative integration frequency of individual donor molecules. Further, the barcode permits identification of the starting components and elements within the donor molecule before transfection.

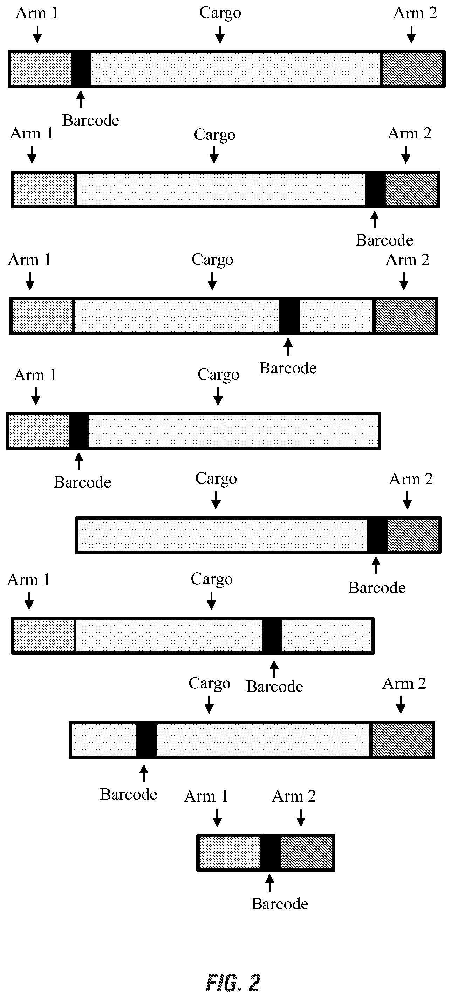

The donor molecules used within the methods described herein can comprise several components, including a cargo, an arm 1, one or more barcodes, and an arm 2 (FIG. 2). In some embodiments, the donor can comprise an arm 1, a barcode, a cargo, and an arm 2. In other embodiments, the donor can comprise a cargo, a barcode, and an arm 2. In other embodiments, the donor can comprise an arm 1, a barcode, and an arm 2.

In one embodiment, the donor molecules described herein can comprise at least one homology sequence and at least one barcode. The donor molecules can comprise one barcode flanked by two homology sequences. The barcode can be between 1 nt and 10 nt, but can be longer (e.g., between 11 nt and 10,000 nt or more). If the desired cargo sequence is small (e.g., between 1 and 100 nucleotides), then the barcode can substitute for the cargo sequence within the donor molecules. The barcode can be the same size as the desired cargo sequence. The barcode can be a smaller size then the desired cargo sequence. The barcode can be a larger size then the desired cargo sequence. For example, if the barcode is one nucleotide, then four donor molecules with different homology arms can be compared. Each of the four donor molecules would have either A, T, G or C as the barcode. If a library of 100 different donors is being compared, then a barcode of at least 4 nucleotides can be used (i.e., 4⁴ = 256 different combinations).

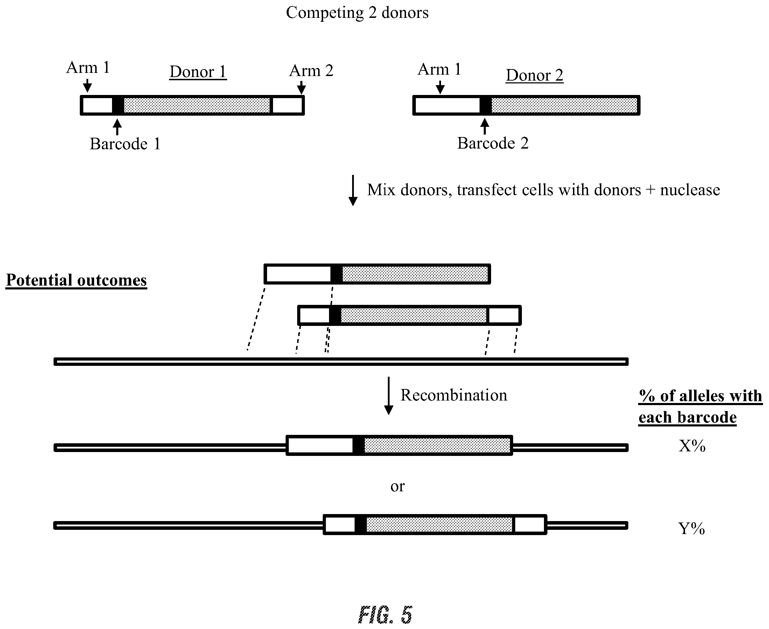

In one embodiment, this document features a method for gene targeting or targeted insertion. The method includes delivering two or more donor molecules to a single cell or a population of cells, wherein the two or more donor molecules have different barcodes (FIG. 5). The two or more donors are delivered to cells along with one or more rare-cutting endonucleases. The donor molecules can integrate into genomic DNA following cleavage by the one or more rare-cutting endonucleases. The frequency of integration of each of the two or more donor molecules can be determined by quantifying the frequency of each barcode present at the target site.

The donor molecules described herein can comprise one or two homology arms. The homology arms can comprise a sequence of DNA homologous to a genomic target site. The homology arms can be a suitable length for participating in homologous recombination with sequence at or near the desired site of integration. The length of each homology arm can be between 50 nt and 10,000 nt or more (e.g., 50 nt, 100 nt, 200 nt, 300 nt, 400 nt, 500 nt, 600 nt, 700 nt, 800 nt, 900 nt, 1,000 nt, 2,000 nt, 3,000 nt, 4,000 nt, 5,000 nt, 6,000 nt, 7,000 nt, 8,000 nt, 9,000 nt, 10,000 nt or more).

The donor molecules described herein can comprise zero, one, two or more target sites for rare-cutting endonucleases. The target sites can be a suitable sequence and length for cleavage by a rare-cutting endonuclease. The target site can be amenable to cleavage by CRISPR systems, TAL effector nucleases, zinc-finger nucleases or meganucleases, or a combination of CRISPR systems, TALE nucleases, zinc finger nucleases or meganucleases, or any other rare-cutting endonuclease. Cutting of the donor molecule by one or more rare-cutting endonucleases can result several outcomes, including a 5' overhang (e.g., Cas12a or TALEN or ZFN), blunt ends (e.g., Cas9), single strand nick (e.g., Cas9 nickase, Cas12a nickase, TALEN nickase or ZFN nickase), or 3' overhang (dual Cas9 nickases, dual Cas12 nickases).

The barcodes described herein can comprise one, two, three, four, five, six, seven, eight, nine, ten, or more nucleic acids. The barcode can be customized for a given library. For example, if the desired library comprises 50 donors, then a barcode of 3 nt (i.e., 64 different combinations) could be sufficient to tag each of the 50 donors with a unique identifier.

In some embodiments, the donor molecules with unique barcodes can be combined to form a library of donors. The library of donors can be a minimum of 2, but can include between 2 and 10,000 donors or more. The donor molecules within the library can be present at equal molar ratios or at equal concentrations. Alternatively, the donors can be present at unequal molar ratios or unequal concentrations. In some embodiments, the donor molecules are all in the same format (e.g., all single-stranded DNA oligonucleotides). In other embodiments, the donor molecules are in different formats (e.g., 50% single-stranded DNA oligonucleotides and 50% double-stranded DNA oligonucleotides).

In some embodiments, donor molecules may be delivered to cells using any suitable method, including but not limited, via transfection, using a non-viral vector, using a viral vector, by chemical means or by exposure to an electric field (e.g., electroporation). Methods of non-viral delivery of nucleic acids include electroporation, lipofection, microinjection, biolistics, virosomes, liposomes, immunoliposomes, polycation or lipid:nucleic acid conjugates, naked DNA, artificial virions, membrane deformation, sonoporation and agent-enhanced uptake of DNA.

In one embodiment, the plurality of donor molecules described herein can comprise different combinations of homology arms, and cargo, and be present on different forms of DNA or vectors, but all should contain one or more unique barcodes if the integration of each donor molecule is to be effectively assessed. By way of example, the plurality of donor molecules can comprise two donor molecules: a first donor molecule with two homology arms, one barcode, and present on a single stranded oligonucleotide; and a second donor molecule with two homology arms which are the same as the first, one barcode different from the first, and present on an AAV vector. By way of another example, the plurality of donor molecules can comprise two donor molecules: a first donor molecule with two homology arms, one barcode, and present on a single-stranded oligonucleotide; and a second donor molecule with two homology arms with different lengths as compared to the first, one barcode different from the first, and present on a single-stranded oligonucleotide. By way of another example, the plurality of donor molecules can comprise two donor molecules: a first donor molecule with two homology arms, one barcode, a cargo, and present on a double-stranded oligonucleotide; and a second donor molecule with two homology arms with different lengths as compared to the first, one barcode different from the first, a cargo the same as the first, and present on a double-stranded oligonucleotide. By way of another example, the plurality of donor molecules can comprise three donor molecules: a first donor molecule with two homology arms, one barcode, a cargo, and present on a double-stranded oligonucleotide; a second donor molecule with two homology arms with different lengths as compared to the first, one barcode different from the first, a cargo the same as the first, and present on a double-stranded oligonucleotide; and a third donor molecule with two homology arms with different lengths as compared to the first and second, one barcode different from the first and second, a cargo the same as the first and second, and present on a double-stranded oligonucleotide.

The donor molecules described herein can be delivered to cell cultures. The cell cultures can be adherent or suspension cell cultures, immortalized cell lines, primary cell lines, or stem cell lines. Additionally, donor molecules can be delivered to cells within organ. The organ can be an animal organ animal. The animal organ can be removed from the animal. If the organ is removed from the animal, the organ can be prepped for transfection. For example, tissue from the organ can be partially digested and maintained within cell culture before transfection. Alternatively, tissue from the organ can be transfected by direct injection with a solution comprising donor molecules. The animal organ can be transfected in vivo. For example, donor molecules can be delivered systemically with carriers such as lipid nanoparticles. The delivery can be achieved using methods such as those described in Finn et al, Cell Reports 22:2227-2235, 2018. Cells from the transfected organ can be assessed for barcode frequencies. Cells or tissue from the organ can be used for nucleic acid purification. In one embodiment, the donor molecules described herein can be delivered to cells from a human patient. The cells can be obtained from a biopsy.

In embodiments, the donor molecules can be the same format of nucleic acids and can comprise structures having a homologous sequence and a barcode. In one embodiment, the donor molecule can have a structure of 5' - [arm 1] - [barcode] - 3'. In another embodiment, the donor molecules can have a structure of 5' - [arm 1] - [barcode] - [arm 2] - 3'. In another embodiment, the donor molecules can have a structure of 5' - [barcode] - [arm 2] - 3'. In another embodiment, the donor molecules can have a structure of 5' - [arm 1] - [cargo] - [barcode] - [arm 2] - 3'. In another embodiment, the donor molecules can have a structure of 5' - [arm 1] - [barcode] - [cargo] - [arm 2] - 3'. In another embodiment, the donor molecules can have a structure of 5' - [arm 1] - [barcode] - [cargo] - 3'. In another embodiment, the donor molecules can have a structure of 5' - [cargo] - [barcode] - [arm 2] - 3'. In another embodiment, the donor molecules can have a structure of 5' - [arm 1] - [barcode 1] - [cargo] - [barcode 2] [arm 2] - 3', wherein barcode 1 and barcode 2 are the same barcode or different barcodes within the same donor, but are different barcodes between two donors with differences in homology arms or format.

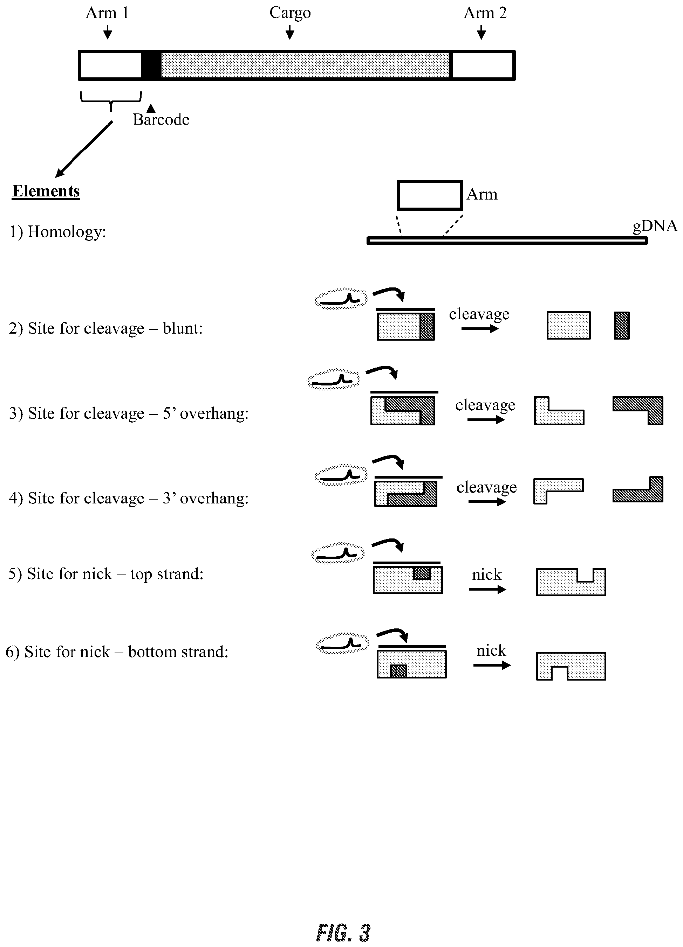

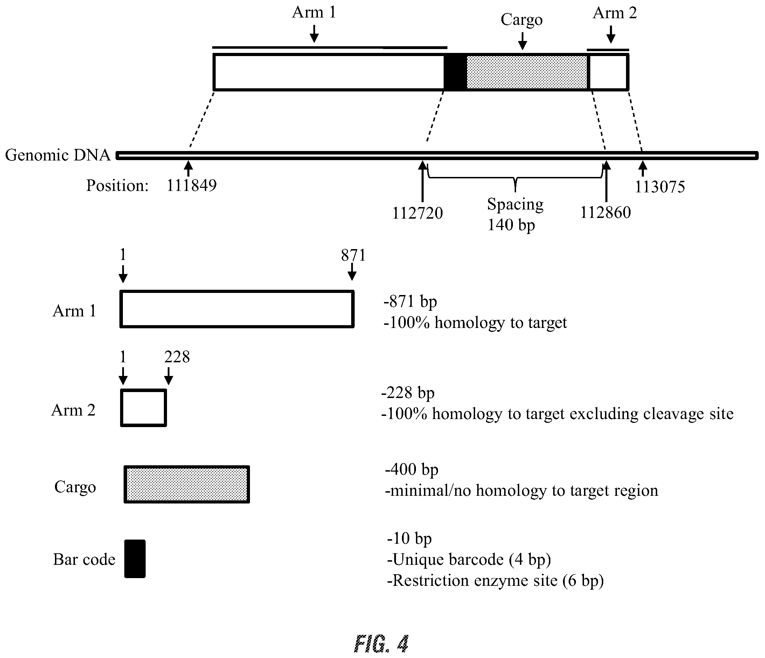

An example of a donor molecule and the properties within the arms of homology, barcode and cargo can be seen in FIGS. 2-4.

In embodiments, the donor molecules can be different formats of nucleic acids and can comprise structures having a homologous sequence and a barcode. In another embodiment, the donor molecules with different formats can also comprise differences in the homology sequence, and differences in barcodes. In one embodiment, two donor molecules can be administered to cells, wherein the donor molecules are harbored on single-stranded oligonucleotides and adeno-associated virus vectors, and both donor molecules have the same homology sequence. In another embodiments, the donor molecules with different formats, the same or different homology sequences and different barcodes can be a combination of single-stranded oligonucleotides, double-stranded oligonucleotides, single-stranded linear DNA, double-stranded linear DNA, single-stranded circular DNA, double-stranded circular DNA, or viral vectors (e.g., adeno-associated virus vectors, adenovirus vectors, lentivirus vectors).

In one embodiment, the barcodes can be detected through sequencing the target locus. Following administration of the plurality of donor molecules, the genomic DNA from the cells can be isolated and subjected to sequencing or PCR/sequencing. The relative barcode frequency can be quantified by determining the number of reads of each barcode. The sequencing can be done by any suitable method, including Maxam-Gilbert sequencing, chain-termination methods, shotgun sequencing, bridge PCR, massively parallel signature sequencing (MPSS), Polony sequencing, 454 pyrosequencing, Illumina (Solexa) sequencing, combinatorial probe anchor synthesis (cPAS), SOLiD sequencing, Ion Torrent semiconductor sequencing, DNA nanoball sequencing, heliscope single molecule sequencing, single molecule real time (SMRT) sequencing, nanopore DNA sequencing, or microfluidic systems.

In another embodiment, the barcodes can be detected through sequencing the RNA. Without being bound by theory, donor molecules which have higher frequencies of integration may result in higher frequencies of the corresponding barcode within the RNA transcripts produced by the target gene. The relative number of barcodes within the RNA transcripts can be used to determine the donor molecule structure with highest efficiencies of integration. Following administration of the plurality of donor molecules, the RNA from the cells can be isolated. The RNA can then be sequenced using any suitable method, including total RNA whole transcriptome sequencing or mRNA sequencing.

The donor molecules and methods provided herein can be used to modify genes encoding proteins within cells. The proteins can include, fibrinogen, prothrombin, tissue factor, Factor V, Factor VII, Factor VIII, Factor IX, Factor X, Factor XI, Factor XII (Hageman factor), Factor XIII (fibrin-stabilizing factor), von Willebrand factor, prekallikrein, high molecular weight kininogen (Fitzgerald factor), fibronectin, antithrombin III, heparin cofactor II, protein C, protein S, protein Z, protein Z-related protease inhibitor, plasminogen, alpha 2-antiplasmin, tissue plasminogen activator, urokinase, plasminogen activator inhibitor-1, plasminogen activator inhibitor-2, glucocerebrosidase (GBA), α-galactosidase A (GLA), iduronate sulfatase (IDS), iduronidase (IDUA), acid sphingomyelinase (SMPD1), MMAA, MMAB, MMACHC, MMADHC (C2orf25), MTRR, LMBRD1, MTR, propionyl-CoA carboxylase (PCC) (PCCA and/or PCCB subunits), a glucose-6-phosphate transporter (G6PT) protein or glucose-6-phosphatase (G6Pase), an LDL receptor (LDLR), ApoB, LDLRAP-1, a PCSK9, a mitochondrial protein such as NAGS (N-acetylglutamate synthetase), CPS1 (carbamoyl phosphate synthetase I), and OTC (ornithine transcarbamylase), ASS (argininosuccinic acid synthetase), ASL (argininosuccinase acid lyase) and/or ARG1 (arginase), and/or a solute carrier family 25 (SLC25A13, an aspartate/glutamate carrier) protein, a UGT1A1 or UDP glucuronsyltransferase polypeptide A1, a fumarylacetoacetate hydrolyase (FAH), an alanine-glyoxylate aminotransferase (AGXT) protein, a glyoxylate reductase/hydroxypyruvate reductase (GRHPR) protein, a transthyretin gene (TTR) protein, an A TP7B protein, a phenylalanine hydroxylase (PAH) protein, an USH2A protein, an ATXN protein, and a lipoprotein lyase (LPL) protein.

The transgene can include sequence for modifying an endogenous gene that harbors a loss-of-function or gain-of-function mutation. The mutation can include those that result in the following genetic diseases: achondroplasia, achromatopsia, acid maltase deficiency, adenosine deaminase deficiency, adrenoleukodystrophy, aicardi syndrome, alpha-1 antitrypsin deficiency, alpha-thalassemia, androgen insensitivity syndrome, pert syndrome, arrhythmogenic right ventricular dysplasia, ataxia telangictasia, barth syndrome, beta-thalassemia, blue rubber bleb nevus syndrome, canavan disease, chronic granulomatous diseases (CGD), cri du chat syndrome, cystic fibrosis, dercum's disease, ectodermal dysplasia, fanconi anemia, fibrodysplasia ossificans progressive, fragile X syndrome, galactosemis, Gaucher's disease, generalized gangliosidoses (e.g., GM1), hemochromatosis, the hemoglobin C mutation in the 6th codon of beta-globin (HbC), hemophilia, Huntington's disease, Hurler Syndrome, hypophosphatasia, Klinefleter syndrome, Krabbes Disease, Langer-Giedion Syndrome, leukocyte adhesion deficiency, leukodystrophy, long QT syndrome, Marfan syndrome, Moebius syndrome, mucopolysaccharidosis (MPS), nail patella syndrome, nephrogenic diabetes insipdius, neurofibromatosis, Neimann-Pick disease, osteogenesis imperfecta, porphyria, Prader-Willi syndrome, progeria, Proteus syndrome, retinoblastoma, Rett syndrome, Rubinstein-Taybi syndrome, Sanfilippo syndrome, severe combined immunodeficiency (SCID), Shwachman syndrome, sickle cell disease (sickle cell anemia), Smith-Magenis syndrome, Stickler syndrome, Tay-Sachs disease, Thrombocytopenia Absent Radius (TAR) syndrome, Treacher Collins syndrome, trisomy, tuberous sclerosis, Turner's syndrome, urea cycle disorder, von Hippel-Landau disease, Waardenburg syndrome, Williams syndrome, Wilson's disease, Wiskott-Aldrich syndrome, X-linked lymphoproliferative syndrome, lysosomal storage diseases (e.g., Gaucher's disease, GM1, Fabry disease and Tay-Sachs disease), mucopolysaccharidosis (e.g. Hunter's disease, Hurler's disease), hemoglobinopathies (e.g., sickle cell diseases, HbC, α-thalassemia, β-thalassemia) and hemophilias. Additional diseases that can be treated by targeted integration include von Willebrand disease, usher syndrome, polycystic kidney disease, spinocerebellar ataxias, spinal and bulbar muscular atrophy, Friedreich's ataxia, myotonic dystrophy type 2, Usher syndrome.

As described herein, the donor molecule can be harbored within a viral or non-viral vector. The vectors can be in the form of circular or linear, double-stranded or single stranded DNA. The donor molecule can be conjugated or associated with a reagent that facilitates stability or cellular update. The reagent can be lipids, calcium phosphate, cationic polymers, DEAE-dextran, dendrimers, polyethylene glycol (PEG) cell penetrating peptides, gas-encapsulated microbubbles or magnetic beads. The donor molecule can be incorporated into a viral particle. The virus can be retroviral, adenoviral, adeno-associated vectors (AAV), herpes simplex, pox virus, hybrid adenoviral vector, epstein-bar virus, lentivirus, or herpes simplex virus.

In certain embodiments, the AAV vectors as described herein can be derived from any AAV. In certain embodiments, the AAV vector is derived from the defective and nonpathogenic parvovirus adeno-associated type 2 virus. All such vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3, 1998; Kearns et al., Gene Ther. 9:748-55, 1996). Other AAV serotypes, including AAV1, AAV2, AAV3, AAV4, AAV5, AAV6, AAV7, AAV8, AAV9 and AAVrh.10 and any novel AAV serotype can also be used in accordance with the present invention. In some embodiments, chimeric AAV is used where the viral origins of the long terminal repeat (LTR) sequences of the viral nucleic acid are heterologous to the viral origin of the capsid sequences. Non-limiting examples include chimeric virus with LTRs derived from AAV2 and capsids derived from AAV5, AAV6, AAV8 or AAV9 (i.e. AAV2/5, AAV2/6, AAV2/8 and AAV2/9, respectively).

The constructs described herein may also be incorporated into an adenoviral vector system. Adenoviral based vectors are capable of very high transduction efficiency in many cell types and do not require cell division. With such vectors, high titer and high levels of expression can been obtained.

The methods and compositions described herein are applicable to any eukaryotic organism in which it is desired to alter the organism through genomic modification. The eukaryotic organisms include plants, algae, animals, fungi and protists. The eukaryotic organisms can also include plant cells, algae cells, animal cells, fungal cells and protist cells.

Exemplary mammalian cells include, but are not limited to, oocytes, K562 cells, CHO (Chinese hamster ovary) cells, HEP-G2 cells, BaF-3 cells, Schneider cells, COS cells (monkey kidney cells expressing SV40 T-antigen), CV-1 cells, HuTu80 cells, NTERA2 cells, NB4 cells, HL-60 cells and HeLa cells, 293 cells (see, e.g., Graham et al. (1977) J. Gen. Virol. 36:59), and myeloma cells like SP2 or NS0 (see, e.g., Galfre and Milstein (1981) Meth. Enzymol. 73(B):3 46). Peripheral blood mononucleocytes (PBMCs) or T-cells can also be used, as can embryonic and adult stem cells. For example, stem cells that can be used include embryonic stem cells (ES), induced pluripotent stem cells (iPSC), mesenchymal stem cells, hematopoietic stem cells, liver stem cells, skin stem cells and neuronal stem cells.

The methods and compositions of the invention can be used in the production of modified organisms. The modified organisms can be small mammals, companion animals, livestock, and primates. Non-limiting examples of rodents may include mice, rats, hamsters, gerbils, and guinea pigs. Non-limiting examples of companion animals may include cats, dogs, rabbits, hedgehogs, and ferrets. Non-limiting examples of livestock may include horses, goats, sheep, swine, llamas, alpacas, and cattle. Non-limiting examples of primates may include capuchin monkeys, chimpanzees, lemurs, macaques, marmosets, tamarins, spider monkeys, squirrel monkeys, and vervet monkeys.

Exemplary plants and plant cells which can be modified using the methods described herein include, but are not limited to, monocotyledonous plants (e.g., wheat, maize, rice, millet, barley, sugarcane), dicotyledonous plants (e.g., soybean, potato, tomato, alfalfa), fruit crops (e.g., tomato, apple, pear, strawberry, orange), forage crops (e.g., alfalfa), root vegetable crops (e.g., carrot, potato, sugar beets, yam), leafy vegetable crops (e.g., lettuce, spinach); vegetative crops for consumption (e.g. soybean and other legumes, squash, peppers, eggplant, celery etc), flowering plants (e.g., petunia, rose, chrysanthemum), conifers and pine trees (e.g., pine fir, spruce); poplar trees (e.g. P. tremula×P. alba); fiber crops (cotton, jute, flax, bamboo) plants used in phytoremediation (e.g., heavy metal accumulating plants); oil crops (e.g., sunflower, rape seed) and plants used for experimental purposes (e.g., Arabidopsis). The methods disclosed herein can be used within the genera Asparagus, Avena, Brassica, Citrus, Citrullus, Capsicum, Cucurbita, Daucus, Erigeron, Glycine, Gossypium, Hordeum, Lactuca, Lolium, Lycopersicon, Malus, Manihot, Nicotiana, Orychophragmus, Oryza, Persea, Phaseolus, Pisum, Pyrus, Prunus, Raphanus, Secale, Solanum, Sorghum, Triticum, Vitis, Vigna, and Zea. The term plant cells include isolated plant cells as well as whole plants or portions of whole plants such as seeds, callus, leaves, and roots. The present disclosure also encompasses seeds of the plants described above wherein the seed has the has been modified using the compositions and/or methods described herein. The present disclosure further encompasses the progeny, clones, cell lines or cells of the transgenic plants described above wherein said progeny, clone, cell line or cell has the transgene or gene construct. Exemplary algae species include microalgae, diatoms, Botryococcus braunii, Chlorella, Dunaliella tertiolecta, Gracileria, Pleurochrysis carterae, Sorgassum and Ulva.

The methods described in this document can include the use of rare-cutting endonucleases for stimulating homologous recombination or non-homologous integration of a donor molecule into genomic target site. The rare-cutting endonuclease can include CRISPR, TALENs, or zinc-finger nucleases (ZFNs). The CRISPR system can include CRISPR/Cas9 or CRISPR/Cas12a (Cpf1). The CRISPR system can include variants which display broad PAM capability (Hu et al., Nature 556, 57-63, 2018; Nishimasu et al., Science DOI: 10.1126, 2018) or higher on-target binding or cleavage activity (Kleinstiver et al., Nature 529:490-495, 2016). The gene editing reagent can be in the format of a nuclease (Mali et al., Science 339:823-826, 2013; Christian et al., Genetics 186:757-761, 2010), nickase (Cong et al., Science 339:819-823, 2013; Wu et al., Biochemical and Biophysical Research Communications 1:261-266, 2014), CRISPR-*FokI* dimers (Tsai et al., Nature Biotechnology 32:569-576, 2014), or paired CRISPR nickases (Ran et al., Cell 154:1380-1389, 2013).

The methods described in this document can be used in a circumstance where it is desired to determine the relative efficiency of two or more donor molecules. For example, patients with Usher syndrome, specifically harboring a c.2299delG, may benefit from correction of the mutation using a donor molecule with or without a nuclease. The methods described herein permit direct comparison of donor molecule efficiencies, thereby permitting the discovery of donor molecules with optimal characteristics. The methods described in this document are useful in any situation where determining donor molecule integration frequency is useful, or for optimizing reagents for therapeutic purposes.

The invention will be further described in the following examples, which do not limit the scope of the invention described in the claims.

### EXAMPLES

### Example 1: Comparing the efficacy of donor molecules targeting the USH2A gene

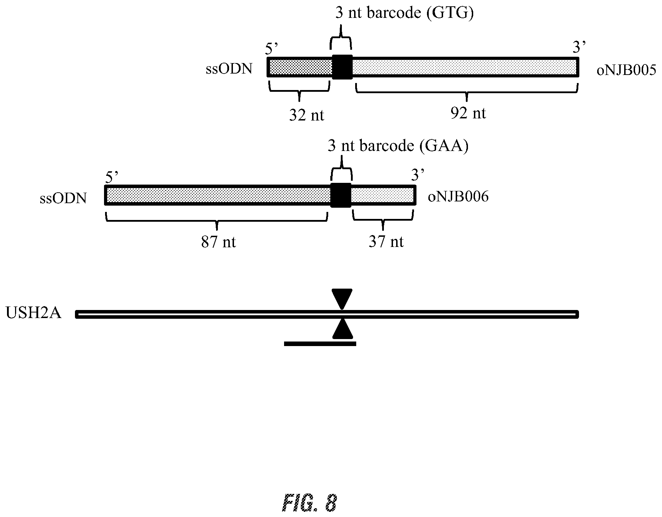

Two single-stranded DNA donor molecules were synthesized with sequence homologous to exon 13 of the USH2A gene (Table 1). Each donor molecule was 127 nt in length but contained different length homology arms (FIG. 8). Each of the two donor molecules contained a unique three nucleotide barcode. The barcodes were designed to be inserted into the gene (i.e., upon recombination, three nucleotides will be added to the gene, with no nucleotides removed). The three nucleotides were positioned in the seed sequence of a Cas9 target site (AATTCTGCAATCCTCACTCT SEQ ID NO: 1) to prevent cleavage of the donor or modified gene.

**Table 1: Donor molecules targeting exon 13 of the USH2A gene**

| Name | Sequence |
|---|---|
| oNJB005 | CATGGCTCAGTGAACAAATTCTGCAATCCTCAGTGCTCT GGGCAGTGTGAGTGCAAAAAAGAAGCCAAAGGACTTCA GTGTGACACCTGCAGAGAAAACTTTTATGGGTTAGATGT CACCAATTGTA (SEQ ID NO: 2) |
| oNJB006 | TAAATTTCTCCGAAGCTTTAATGATGTTGGATGTGAGCC CTGCCAGTGTAACCTCCATGGCTCAGTGAACAAATTCTG CAATCCTCAGAACTCTGGGCAGTGTGAGTGCAAAAAAG AAGCCAAAGGA (SEQ ID NO: 3) |

Transfection was performed using immortalized HEK293T cells. HEK293T cells were maintained at 37°C and 5% CO2 in DMEM high supplemented with 10% fetal bovine serum (FBS). HEK293T cells were transfected with equal molar concentrations of the donor molecules along with the Cas9 nuclease. In samples transfected with one donor molecule, the donor was delivered at 4 uM concentration. In samples transfected with two donor molecules, the donors were delivered at 2 uM concentrations each. Transfections were performed using electroporation. The frequency of each barcode was determined by deep sequencing. Approximately 20,000 reads were obtained for each sample.

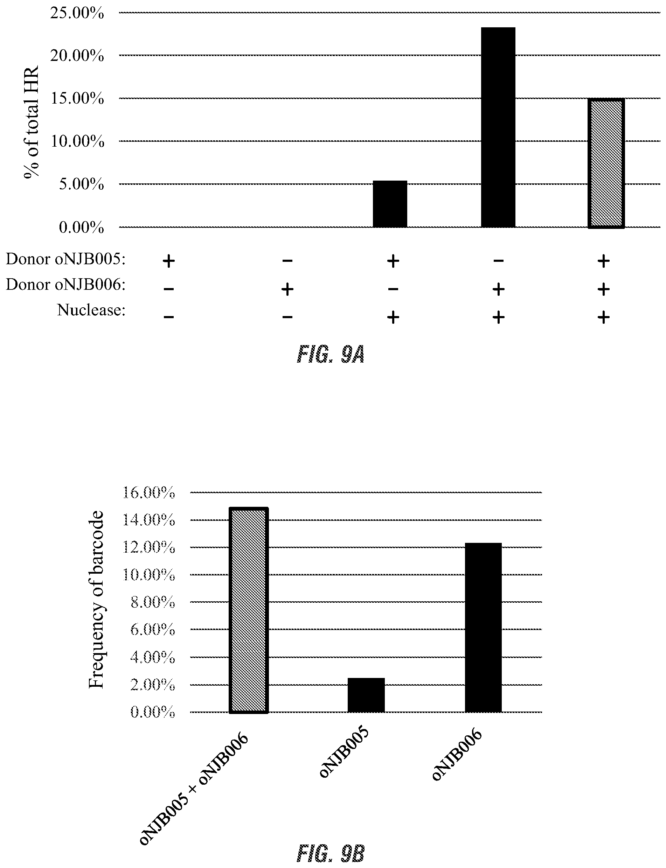

In samples delivered oNJB005 or oNJB006 alone, no NHEJ or gene targeting was observed (FIG. 9A, columns 1 and 2). In samples delivered oNJB005 and the nuclease, 91.3% of the cells contained a modification at the target site (NHEJ + HR) and 5.36% contained the barcode from oNJB005. In samples delivered oNJB006 and the nuclease, 94.75% of the cells contained a modification at the target site (NHEJ + HR) and 23.27% contained the barcode from oNJB006. In samples delivered oNJB005 and oNJB006 and the nuclease, 94.29% of the cells contained a modification at the target site (NHEJ + HR) and 14.82% contained the barcode from oNJB006 or oNJB005 (combined HR).

To determine the relative frequency of each barcode in the sample delivered both donors, the percentage of each barcode was determined (FIG. 9B). Within the 14.82% of cells with an HR event, 2.5% comprised the barcode from oNJB005, while the remaining 12.32% comprised the barcode from oNJB006. The results from the competition assay indicate donor oNJB005 outperformed donor oNJB006 by approximately 4.9X. In comparison, the results from individual tubes indicate donor oNJB005 outperformed donor oNJB006 by approximately 4.3X.

### Example 2: Comparing the efficacy of donor molecules targeting the HBB gene

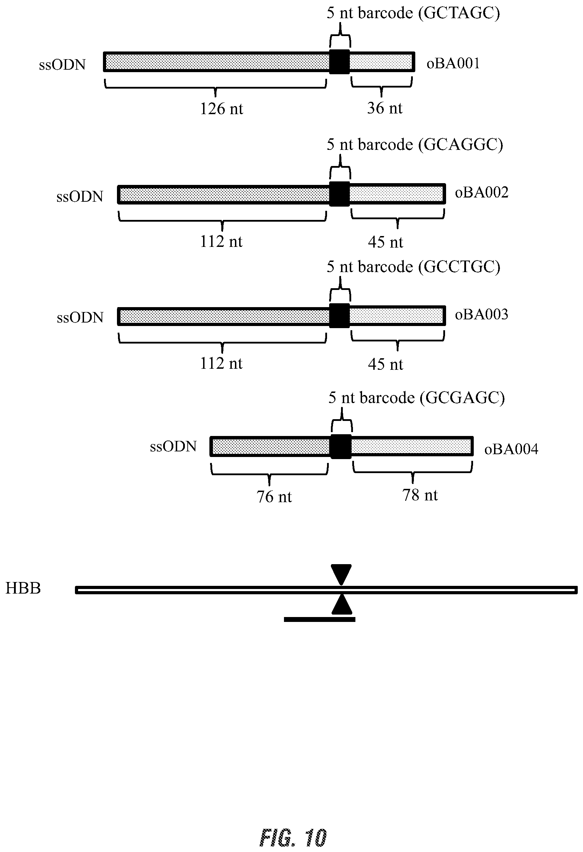

Four single-stranded DNA donor molecules were synthesized with sequence homologous to intron 1 of the HBB gene (Table 2). Each of the donor molecules contained a unique six nucleotide barcode (FIG. 10). The barcodes were designed to be inserted into the gene (i.e., upon recombination, the six nucleotides will be added to the gene, with no nucleotides being removed). The six nucleotides were positioned in the seed sequence of a Cas9 target site (GGGTGGGAAAATAGACCAAT SEQ ID NO: 4) to prevent cleavage of the donor or modified gene. Notably, oNJB002 was designed to be identical to oNJB003, outside of 2 nucleotides within the barcodes (GCAGGC compared to GCCTGC). Both comprised the same 112 nucleotide left homology arm and 45 nucleotide right homology arm.

**Table 2: Donor molecules targeting intron 1 of the HBB gene**

| Name | Sequence |
|---|---|
| oN.IB001 | GGTGAGGCCCTGGGCAGGTTGGTATCAAGGTTACAAGACAGG TTTAAGGAGACCAATAGAAACTGGGCATGTGGAGACAGAGA AGACTCTTGGGTTTCTGATAGGCACTGACTCTCTCTGCCTATT GCTAGCGGTCTATTTTCCCACCCTTAGGCTGCTGGTGGTCTA (SEQ ID NO: 5) |
| oNJB002 | CAGGTTGGTATCAAGGTTACAAGACAGGTTTAAGGAGACCAA TAGAAACTGGGCATGTGGAGACAGAGAAGACTCTTGGGTTTC TGATAGGCACTGACTCTCTCTGCCTATTGCAGGCGGTCTATTT TCCCACCCTTAGGCTGCTGGTGGTCTACCCTTGGAC (SEQ ID NO: 6) |
| oNJB003 | CAGGTTGGTATCAAGGTTACAAGACAGGTTTAAGGAGACCAA TAGAAACTGGGCATGTGGAGACAGAGAAGACTCTTGGGTTTC TGATAGGCACTGACTCTCTCTGCCTATTGCCTGCGGTCTATTTT CCCACCCTTAGGCTGCTGGTGGTCTACCCTTGGAC (SEQ ID NO: 7) |
| oNJB004 | GACCAATAGAAACTGGGCATGTGGAGACAGAGAAGACTCTTG GGTTTCTGATAGGCACTGACTCTCTCTGCCTATTGCGAGCGGT |
| | CTATTTTCCCACCCTTAGGCTGCTGGTGGTCTACCCTTGGACC CAGAGGTTCTTTGAGTCCTTTGGGGATCTGTC (SEQ ID NO: 8) |

Transfection was performed using immortalized HEK293T cells. HEK293T cells were maintained at 37°C and 5% CO2 in DMEM high supplemented with 10% fetal bovine serum (FBS). HEK293T cells were transfected with equal molar concentrations of the donor molecules along with the Cas9 nuclease. In samples transfected with one donor molecule, the donor was delivered at 4 uM concentration. In samples transfected with four donor molecules, the donors were delivered at 1 uM concentrations each. Transfections were performed using electroporation. The frequency of each barcode was determined by deep sequencing. Approximately 50,000 reads were obtained for each sample.

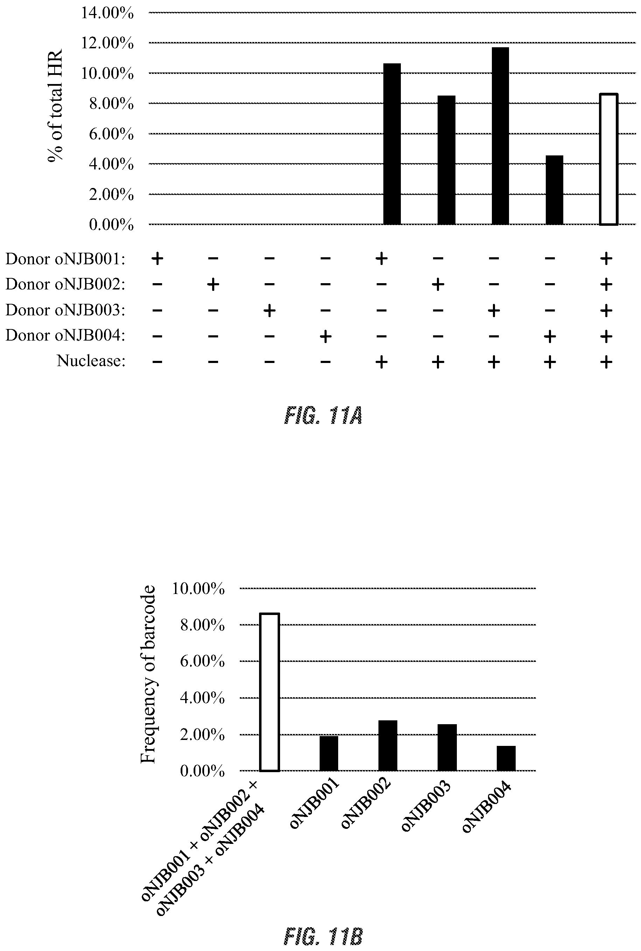

In samples delivered oNJB001, oNJB002, oNJB003 or oNJB004 alone, no NHEJ or gene targeting was observed (FIG. 11A, columns 1 - 4). In samples delivered oNJB001 and the nuclease, 82.85% of the cells contained a modification at the target site (NHEJ + HR) and 10.64% contained the barcode from oNJB001. In samples delivered oNJB002 and the nuclease, 81.64% of the cells contained a modification at the target site (NHEJ + HR) and 8.50% contained the barcode from oNJB002. In samples delivered oNJB003 and the nuclease, 85.84% of the cells contained a modification at the target site (NHEJ + HR) and 11.70% contained the barcode from oNJB003. In samples delivered oNJB004 and the nuclease, 79.83% of the cells contained a modification at the target site (NHEJ + HR) and 4.55% contained the barcode from oNJB004.

In samples delivered oNJB001, oNJB002, oNJB003 and oNJB004 and the nuclease, 83.57% of the cells contained a modification at the target site (NHEJ + HR) and 8.61% contained the barcode from oNJB001, oNJB002, oNJB003 or oNJB004 (combined HR).

To determine the relative frequency of each barcode in the sample delivered all four donors, the % of each barcode was determined (FIG. 11B). Within the 8.61% of cells with an HR event, 1.91% comprised the barcode from oNJB001, 2.78% comprised the barcode from oNJB002, 2.56% comprised the barcode from oNJB003, and 1.36% comprised the barcode from oNJB004.

The results from the individual tubes indicated that donor oNJB003 outperformed the other donors, with oNJB001 performing the closest to oNJB003. It was somewhat unexpected to see the difference in HR between oNJB002 and oNJB003, given they were identical donors except for two nucleotides within the barcode. This may be due to i) differences in NHEJ activity by the nuclease (compare 81.64% for oNJB002 to 85.84% for oNJB003), ii) variability in the technical conditions for transfection, or iii) efficiency differences caused by the nucleotide changes in the barcode.

Contrariwise, the results from the competition assay indicate that donor oNJB002 outperformed the other donors, with oNJB003 having close editing efficiencies with oNJB002. Comparing the results from individual tubes to the competition assay, there were similarities, including donor oNJB004 performing the worst; however, there was also significant differences, including donor oNJB002 and oNJB003 being closest in editing efficiencies in the competition assay. This difference may indicate that i) the efficiency differences caused by changes within the barcode are minimal, and ii) the competition assay may be a more accurate means to test donor molecule efficiencies as compared to individually testing donors, as the variability with the nuclease and technical conditions with the transfection are reduced.

### Example 3: Comparing the efficacy of donor molecules targeting the PPP1R12C gene

Two single-stranded DNA donor molecules were synthesized with sequence homologous to the PPP1R12C gene (Table 3). Both donor molecules had symmetrical homology arms, however, one donor was 123 nt while the other was 63 nt. Each of the donor molecules contained a unique three nucleotide barcode. The barcodes were designed to be inserted into the gene (i.e., upon recombination, the three nucleotides will be added to the gene, with no nucleotides being removed). The three nucleotides were positioned in the seed sequence of a Cas9 target site (GGGGCCACTAGGGACAGGAT SEQ ID NO: 9).

**Table 3: Donor molecules targeting the PPP1R12C gene**

| Name | Sequence |
|---|---|
| oNJB007 | AGGCCTAAGGATGGGGCTTTTCTGTCACCA GAAATCCTGTCCCTAGTGGCCCCACTGTGG GGT (SEQ ID NO: 10) |
| oNJB008 | AGACCCAATATCAGGAGACTAGGAAGGAGGAGGCCTAA GGATGGGGCTTTTCTGTCACCAGCTATCCTGTCCCTAGT GGCCCCACTGTGGGGTGGAGGGGACAGATAAAAGTACC CAGAACCA (SEQ ID NO: 11) |

Transfection was performed using immortalized HEK293T cells. HEK293T cells were maintained at 37°C and 5% CO2 in DMEM high supplemented with 10% fetal bovine serum (FBS). HEK293T cells were transfected with equal molar concentrations of the donor molecules along with the Cas9 nuclease. Transfections were performed using Lipofectamine. The frequency of each barcode is determined by deep sequencing.

### Example 4: Comparing the efficacy of donor molecules targeting the USH2A gene

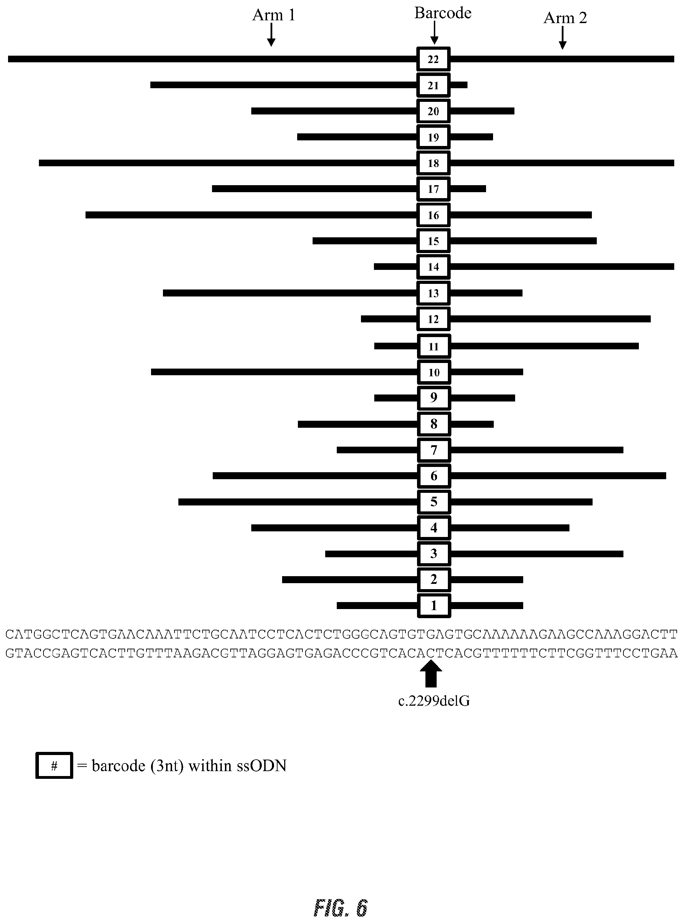

A library of single-stranded oligos are synthesized with sequence homologous to exon 13 of the USH2A gene. Each donor molecules also comprises a unique three nucleotide barcode. The three nucleotide barcodes were designed to be inserted into the gene (i.e., upon recombination, the three nucleotides will be added to the gene, with no nucleotides being removed). A total of 22 donors are synthesized. The donors are mixed together in an equal molar ratio to create a donor library pool to be used in subsequent transfections (FIG. 6).

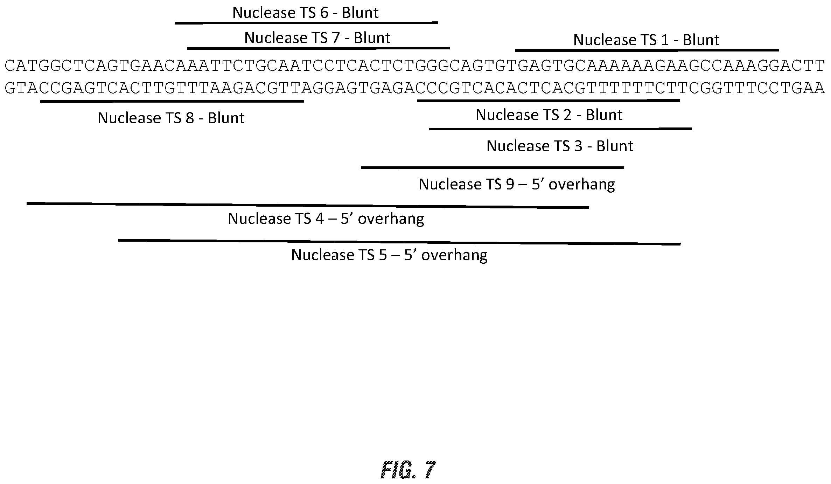

A series of nucleases are designed to target sequence at or near the desired site of integration (FIG. 7). Each of the nucleases are tested individually with the donor library pool (Table 4).

**Table 4: Transfections using individual nucleases together with a donor library**

| Transfection Number | Nuclease target site (TS) | Donor library |
|---|---|---|
| 1 | TS1 | ssODNs 1-22 |
| 2 | TS2 | ssODNs 1-22 |
| 3 | TS3 | ssODNs 1-22 |
| 4 | TS4 | ssODNs 1-22 |
| 5 | TS5 | ssODNs 1-22 |
| 6 | TS6 | ssODNs 1-22 |
| 7 | TS7 | ssODNs 1-22 |
| 8 | TS8 | ssODNs 1-22 |
| 9 | TS9 | ssODNs 1-22 |

Transfection is performed using immortalized HEK293 cells. HEK293 cells are maintained at 37°C and 5% CO2 in DMEM high glucose without L-glutamine without sodium pyruvate medium supplemented with 10% fetal bovine serum (FBS) and 1 % penicillin-streptomycin (PS) solution 100X. HEK293 cells are transfected with each of the plasmid constructs and combinations thereof using Lipofectamine 3000. To minimize the presence of residual oligonucleotides in the samples, cells are passaged multiple times before genomic DNA extraction. DNA is extracted and assessed for mutations and targeted insertions within the USH2A gene. Primers are designed to capture a 400 bp sequence harboring the target site but outside the arms of the donor molecules. The 400 bp amplicons are deep sequenced and the frequency of each barcode is calculated.

### Example 5: Comparing the efficacy of single-stranded donor molecules to double-stranded donor molecules

A single-stranded DNA donor molecule was synthesized with sequence homologous to exon 13 of the USH2A gene (oNJB005). A second double-stranded DNA donor molecule was synthesized with sequence identical to the single-stranded donor, except for changes within the barcode.

Transfection is performed using immortalized HEK293 cells. HEK293 cells are maintained at 37°C and 5% CO2 in DMEM high glucose without L-glutamine without sodium pyruvate medium supplemented with 10% fetal bovine serum (FBS) and 1% penicillin-streptomycin (PS) solution 100X. HEK293 cells are transfected with each of the plasmid constructs and combinations thereof using Lipofectamine 3000. To minimize the presence of residual oligonucleotides in the samples, cells are passaged multiple times before genomic DNA extraction. DNA is extracted and assessed for mutations and targeted insertions within the USH2A gene. Primers are designed to capture a 400 bp sequence harboring the target site but outside the arms of the donor molecules. The 400 bp amplicons are deep sequenced and the frequency of each barcode is calculated.

### Example 6: Comparing the efficacy of donor molecules in cells within organs

Four single-stranded DNA donor molecules are synthesized with sequence homologous to the GLA gene in mice. Each of the donor molecules contains a unique six nucleotide barcode. A corresponding Cas9 nuclease is designed to cleave the target GLA gene. The donor molecules are mixed at equal molar ratios along with the Cas9 and gRNA in RNA format. The gene editing reagents are then combined with lipid nanoparticles and delivered to mice by tail vein injection.

Four days post injection, the liver is removed, and DNA is extracted and assessed for mutations and targeted insertions within the GLA gene. Primers are designed to capture a 400 bp sequence harboring the target site but outside the arms of the donor molecules. The 400 bp amplicons are deep sequenced and the frequency of each barcode is calculated.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A method of identifying the frequency of donor molecule integration into genomic DNA in cells comprising:
(i) exposing the cells to a plurality of donor molecules and a rare-cutting endonuclease, wherein each donor molecule comprises (i) a homology sequence, and (ii) at least one barcode,
wherein the homology sequence comprises a sequence that is homologous to a target locus within the genomic DNA, wherein the homology sequence for each donor molecule is different from the homology sequences of other donor molecules, wherein the plurality of donor molecules comprises homologous sequences with homology to a genomic DNA sequence within the same gene; and
wherein the at least one barcode for each donor molecule is different from the barcodes for other donor molecules;
(ii) sequencing the resulting barcoded genomic DNA or RNA of the resulting cells; and,
(iii) determining the frequency of integration of each barcode into the genomic DNA of the resulting cells thereby identifying the frequency of donor molecule integration into the genomic DNA of the resulting cells.

2. The method of claim 1, wherein the homology sequence for each donor molecule comprises at least one homology arm.

3. The method of claim 1, wherein the donors additionally comprise a cargo sequence.

4. The method of claim 3, wherein the cargo sequences are the same.

5. The method of claim 1, wherein the cells are exposed to an equal molar ratio or equal concentration of each of the donor molecules within the plurality of donor molecules.

6. The method of claim 1, wherein the plurality of donor molecules comprises at least two donor molecules.

7. The method of claim 6, wherein the plurality of donor molecules comprises at least ten donor molecules.

8. The method of claim 7, wherein the plurality of donor molecules comprises at least one hundred donor molecules.

9. The method of claim 1, wherein the rare-cutting endonuclease is selected from a CRISPR nuclease and a zinc-finger nuclease.

10. The method of claim 1, wherein the genomic DNA is from a eukaryotic cell.

11. The method of claim 1, wherein the donor molecule format is selected from single-stranded oligonucleotides, double-stranded oligonucleotides, single-stranded linear DNA, double-stranded linear DNA, single-stranded circular DNA, double-stranded circular DNA.

12. A composition comprising a plurality of donor molecules, wherein
each donor molecule comprises (i) a homology sequence, and (ii) at least one barcode, wherein the homology sequence comprises a sequence that is homologous to a target locus within a genome wherein the plurality of donor molecules comprises homologous sequences with homology to a genomic DNA sequence within the same gene,
wherein the homology sequence for each donor molecule is different from the homology sequences of other donor molecules; and
wherein the at least one barcode for each donor molecule is different from the barcodes for other donor molecules.

13. The method of claim 1, further comprising the following step:
(iv) identifying the donor molecule structure with the highest frequency of integration as the optimal donor molecule structure for integration into the genomic DNA of cells.

## Patentansprüche

1. Verfahren zur Identifizierung der Häufigkeit der Integration von Donormolekülen in genomische DNA in Zellen, umfassend:
(i) Aussetzen der Zellen einer Vielzahl von Donormolekülen und einer selten schneidenden Endonuklease, wobei jedes Donormolekül (i) eine Homologiesequenz und (ii) mindestens einen Barcode umfasst,
wobei die Homologiesequenz eine Sequenz umfasst, die zu einem Ziellocus innerhalb der genomischen DNA homolog ist, wobei sich die Homologiesequenz jedes Donormoleküls von den Homologiesequenzen der anderen Donormoleküle unterscheidet, wobei die Vielzahl von Donormolekülen Homologiesequenzen umfasst, die zu einer genomischen DNA-Sequenz innerhalb desselben Gens homolog sind, und
wobei sich der mindestens eine Barcode jedes Donormoleküls von den Barcodes der anderen Donormoleküle unterscheidet;
(ii) Sequenzieren der resultierenden mit Barcodes versehenen genomischen DNA oder RNA der resultierenden Zellen; und
(iii) Bestimmen der Integrationshäufigkeit jedes Barcodes in die genomische DNA der resultierenden Zellen, wodurch die Häufigkeit der Integration der Donormoleküle in die genomische DNA der resultierenden Zellen identifiziert wird.

2. Verfahren nach Anspruch 1, wobei die Homologiesequenz jedes Donormoleküls mindestens einen Homologiearm umfasst.

3. Verfahren nach Anspruch 1, wobei die Donor ferner eine Frachtsequenz umfassen.

4. Verfahren nach Anspruch 3, wobei die Frachtsequenzen identisch sind.

5. Verfahren nach Anspruch 1, wobei die Zellen einem gleichen molaren Verhältnis oder derselben Konzentration jedes Donormoleküls innerhalb der Vielzahl von Donormolekülen ausgesetzt werden.

6. Verfahren nach Anspruch 1, wobei die Vielzahl von Donormolekülen mindestens zwei Donormoleküle umfasst.

7. Verfahren nach Anspruch 6, wobei die Vielzahl von Donormolekülen mindestens zehn Donormoleküle umfasst.

8. Verfahren nach Anspruch 7, wobei die Vielzahl von Donormolekülen mindestens einhundert Donormoleküle umfasst.

9. Verfahren nach Anspruch 1, wobei die selten schneidende Endonuklease aus einer CRISPR-Nuklease und einer Zinkfinger-Nuklease ausgewählt ist.

10. Verfahren nach Anspruch 1, wobei die genomische DNA aus einer eukaryotischen Zelle stammt.

11. Verfahren nach Anspruch 1, wobei das Format des Donormoleküls aus einzelsträngigen Oligonukleotiden, doppelsträngigen Oligonukleotiden, einzelsträngiger linearer DNA, doppelsträngiger linearer DNA, einzelsträngiger zirkulärer DNA, doppelsträngiger zirkulärer DNA ausgewählt ist.

12. Zusammensetzung, umfassend eine Vielzahl von Donormolekülen, wobei
jedes Donormolekül (i) eine Homologiesequenz und (ii) mindestens einen Barcode umfasst, wobei die Homologiesequenz eine Sequenz umfasst, die zu einem Ziellocus innerhalb eines Genoms homolog ist, wobei die Vielzahl von Donormolekülen Homologiesequenzen umfasst, die zu einer genomischen DNA-Sequenz innerhalb desselben Gens homolog sind,
wobei sich die Homologiesequenz jedes Donormoleküls von den Homologiesequenzen der anderen Donormoleküle unterscheidet, und
wobei sich der mindestens eine Barcode jedes Donormoleküls von den Barcodes der anderen Donormoleküle unterscheidet.

13. Verfahren nach Anspruch 1, ferner umfassend den folgenden Schritt:
(iv) Identifizieren der Donormolekülstruktur mit der höchsten Integrationshäufigkeit als optimale Donormolekülstruktur für die Integration in die genomische DNA von Zellen.

## Revendications

1. Procédé d'identification de la fréquence d'intégration de molécules donneuses dans l'ADN génomique dans les cellules, comprenant :
(i) l'exposition des cellules à une pluralité de molécules donneuses et à une endonucléase à coupure rare, dans lequel chaque molécule donneuse comprend (i) une séquence d'homologie, et (ii) au moins un code-barres,
dans lequel la séquence d'homologie comprend une séquence qui est homologue à un locus cible dans le DNA génomique, dans lequel la séquence d'homologie pour chaque molécule donneuse est différente des séquences d'homologie des autres molécules donneuses, dans lequel la pluralité de molécules donneuses comprend des séquences homologues présentant une homologie avec une séquence d'ADN génomique dans le même gène ; et
dans lequel l'au moins un code-barres pour chaque molécule donneuse est différent du code-barres pour les autres molécules donneuses ;
(ii) le séquençage de l'ADN ou ARN génomique à code-barres résultant des cellules résultantes ; et,
(iii) la détermination de la fréquence d'intégration de chaque code-barres dans l'ADN génomique des cellules résultantes identifiant ainsi la fréquence d'intégration de molécule donneuse dans l'ADN génomique des cellules résultantes.

2. Procédé selon la revendication 1, dans lequel la séquence d'homologie pour chaque molécule donneuse comprend au moins un bras d'homologie.

3. Procédé selon la revendication 1, dans lequel les donneuses comprennent en outre une séquence cargo.

4. Procédé selon la revendication 3, dans lequel les séquences cargo sont les mêmes.

5. Procédé selon la revendication 1, dans lequel les cellules sont exposées à un rapport molaire égal ou à une concentration égale de chacune des molécules donneuses de la pluralité de molécules donneuses.

6. Procédé selon la revendication 1, dans lequel la pluralité de molécules donneuses comprend au moins deux molécules donneuses.

7. Procédé selon la revendication 6, dans lequel la pluralité de molécules donneuses comprend au moins dix molécules donneuses.

8. Procédé selon la revendication 7, dans lequel la pluralité de molécules donneuses comprend au moins cent molécules donneuses.

9. Procédé selon la revendication 1, dans lequel l'endonucléase à coupure rare est choisie parmi une nucléase CRISPR et une nucléase à doigt de zinc.

10. Procédé selon la revendication 1, dans lequel l'ADN génomique provient d'une cellule eucaryote.

11. Procédé selon la revendication 1, dans lequel le format de molécule donneuse est choisi parmi des oligonucléotides monobrin, des oligonucléotides à double brin, des ADN linéaires monobrin, des ADN linéaires à double brin, des ADN circulaires monobrin, des ADN circulaires à double brin.

12. Composition comprenant une pluralité de molécules donneuses, dans laquelle
chaque molécule donneuse comprend (i) une séquence d'homologie, et (ii) au moins un code-barres, dans laquelle la séquence d'homologie comprend une séquence qui est homologue à un locus cible dans un génome dans laquelle la pluralité de molécules donneuses comprend des séquences homologues présentant une homologie avec une séquence d'ADN génomique dans le même gène,
dans laquelle la séquence d'homologie pour chaque molécule donneuse est différente des séquences d'homologie des autres molécules donneuses ; et
dans laquelle l'au moins un code-barres pour chaque molécule donneuse est différent des codes-barres pour les autres molécules donneuses.

13. Procédé selon la revendication 1, comprenant en outre l'étape suivante :
(iv) l'identification de la structure de molécule donneuse avec la fréquence d'intégration la plus élevée en tant que structure de molécule donneuse optimale pour intégration dans l'ADN génomique de cellules.
